(19) Europäisches Patentamt
European Patent Office
Office européen des brevets

(11) Publication number: **0 212 867**
**B1**

(12) **EUROPEAN PATENT SPECIFICATION**

(45) Date of publication of the patent specification: **10.05.89**

(51) Int. Cl.⁴: **C 07 H 17/04,** A 61 K 31/70, C 12 P 17/08, C 12 P 19/62 // A23K1/17 ,(C12P17/08, C12R1:365),(C12P19/62, C12R1:365)

(21) Application number: **86305765.9**

(22) Date of filling: **25.07.86**

(54) **Antihelmintic avermectin derivatives produced by the fermentation of a microorganism.**

(30) Priority: **29.07.85 US 759780**

(43) Date of publication of application: **04.03.87 Bulletin 87/10**

(45) Publication of the grant of the patent: **10.05.89 Bulletin 89/19**

(84) Designated Contracting States: **CH DE FR GB IT LI NL**

(56) References cited:
**EP-A-0 065 403**
**EP-A-0 074 758**
**EP-A-0 194 125**

(73) Proprietor: **MERCK & CO. INC., 126, East Lincoln Avenue P.O. Box 2000, Rahway New Jersey 07065- 0900 (US)**

(72) Inventor: **Goegelman, Robert T., 437 Academy Terrace, Linden New Jersey 07036 (US)**
Inventor: **Inamine, Edward S., 376 Russell Avenue, Rahway New Jersey 07065 (US)**
Inventor: **White, Raymond F., 12 Becket Road, Englishtown New Jersey 07726 (US)**

(74) Representative: **Crampton, Keith John Allen, D YOUNG & CO 10 Staple Inn, London WC1V 7RD (GB)**

**EP 0 212 867 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

LIBER, STOCKHOLM 1989

**Description**

This invention is concerned with derivatives of avermectin compounds.

Avermectin compounds are disclosed in U.S. Patent Specification US-A-4 310 519 and dihydro avermectin compounds are disclosed in U.S. Patent Specification US-A-4 199 569.

It has been discovered that novel macrocyclic lactones are produced by the fermentation, with avermectin B1a, avermectin B1b or 22, 23-dihydro avermectin B1a as substrates, of a nutrient medium with a strain of the microorganism Nocardia autotrophica MA-6181, which is a known microorganism, in the culture collection of Merck & Co. Inc., Rahway, New Jersey, USA, and is available from the American Type Culture Collection, of 12301 Parklawn Drive, Rockville, Md. 20852, as ATCC 35203. It has also been found that these novel lactones have antiparasitic and insecticidal activity, in particular anthelmintic, acaricidal and nematocidal activity.

In accordance with this invention, a novel substance is prepared by growing under controlled conditions in a suitable aqueous nutrient medium with, as a substrate, avermectin B1a, avermectin B1b or 22, 23-dihydro avermectin B1a, a known strain of microorganism, Nocardia autotrophica sub. sp. canberrica ATCC 35203. The compounds obtained by fermentation are recovered in substantially pure form as described herein.

Aqueous media such as those used for the production of many antibiotic substances are suitable for use in the process of the present invention. Such nutrient media contain sources of carbon and nitrogen assimilable by the microorganism and generally low levels of inorganic salts. They may also contain traces of metals necessary for the growth of the microorganisms, and production of the desired compounds. These are usually present in sufficient concentrations in the complex sources of carbon and nitrogen, which may be used as nutrient sources, but can, of course, be added separately to the medium if desired.

In general, carbohydrates such as sugars, for example dextrose, sucrose, maltose, lactose, dextran and cerelose, corn meal, oat flour and starches are suitable sources of assimilable carbon in the nutrient media. The exact quantity of the carbon source will depend, in part, upon the other ingredients in the medium, but from 0.5 to 5 % by weight, based on the medium, is usually satisfactory. These carbon sources can be used individually or several may be combined in the same medium.

Various nitrogen sources such as yeast hydrolysates, yeast autolysates, yeast cells, tomato paste, corn meal, oat flour, soybean meal, casein hydrolysates, yeast extracts, corn steep liquors, distillers' solubles, cottonseed meal and meat extract are readily assimilated by Nocardia autotrophica ATCC 35203 in the production of the novel compounds. The various sources of nitrogen can be used alone or in combination in amounts ranging from 0.2 to 6 % by weight of the medium.

Among the nutrient inorganic salts that can be incorporated in the culture media are the customary salts capable of yielding ions such as those of sodium, potassium, magnesium, ammonium, calcium, phosphate, sulfate, chloride and carbonate, as well as trace metals such as cobalt and manganese.

The media described below and in the Examples are merely illustrative and not limitative of those that can be used.

The following are Examples of media suitable for growing strains of Nocardia autotrophica ATCC 35203.

**Medium 1**

| | |
|---|---|
| Dextrose | 1.0 g |
| Dextrin (Fisher) | 10.0 g |
| Beef Extract (Difco) | 3.0 g |
| Yeast Autolysate (Ardamine pH, Yeast prod.) | 5.0 g |
| NZ Amine Type E (Sheffield) | 5.0 g |
| $MgSO_4 \cdot 7H_2O$ | 0.05 g |
| Phosphate Buffer | 2 ml |
| $CaCO_3$ | 0.5 g |
| $dH_2O$ | 1000 ml |
| pH 7.0 - 7.2 | |

| | | |
|---|---|---|
| Phosphate Buffer: | $KH_2PO_4$ | 91.0 g |
| | $Na_2HPO_4$ | 95.0 g |
| | $dH_2O$ | 1000 ml |
| | pH 7.0 | |

**Medium 2**

| | |
|---|---|
| Yeast Extract (Difco) | 4.0 g |
| Malt Extract (Difco) | 10.0 g |
| Dextrose | 4.0 g |
| $dH_2O$ | 1000 ml |
| Agar | 20 g |
| pH 7.2 | |

**Medium 3**

**Basal**

| | |
|---|---|
| Sucrose | 103 g |
| $K_2SO_4$ | 0.25 g |
| Glucose | 10 g |
| L-Asparagine | 1.8 g |
| Casamino Acids (Difco) | 0.1 g |
| $MgCl_2 \cdot 6H_2O$ | 10.12 g |
| Trace Element Mix A | 2 ml |
| $dH_2O$ | to 700 ml |
| Agar | 22.0 g |

Post-sterilization additions, per 700 ml Basal:

100 ml of $CaCl_2$ solution (29.5 g/1000 ml $dH_2O$)

100 ml of $KH_2PO_4$ solution (0.5 g/1000 ml $dH_2O$)

100 ml of Tes solution (0.3 g Tris HCl + 0.1 g EDTA + 0.14 g NaCl in 1000 ml $dH_2O$, adjust to pH 8.0)

**Trace Element Mix A Composition:**

| | |
|---|---|
| $Fe(SO_4)3 \cdot 7H_2O$ | 250 mg |
| $MnCl_2 \cdot 4H_2O$ | 500 mg |
| $CuCl_2 \cdot 2H_2O$ | 25 mg |
| $CaCl_2 \cdot 2H_2O$ | 1000 mg |
| $H_3BO_3$ | 50 mg |
| $(NH_4)_6Mo_7O_{24} \cdot 4H_2O$ | 20 mg |
| $ZnSO_4 \cdot 7H_2O$ | 100 mg |
| $Co(NO_3)_2 \cdot 6H_2O$ | 20 mg |
| 0.1N HCl | 1000 ml |

**Medium 4**

| | |
|---|---|
| Dextrin (Fisher) | 40 g |
| Distillers Solubles (Grain Processing Corp.) | 7 g |
| Yeast Extract (Oxoid) | 5 g |
| $CoCl_2 \cdot 6H_2O$ | 50 mg |
| $dH_2O$ | 1000 ml |
| | pH 7.3 |

**Medium 5**

| | |
|---|---|
| Dextrose | 45 g |
| Peptonized Milk (Sheffield) | 24 g |
| Ardamine pH (Yeast Products, Inc.) | 2.5 g |
| Polyglycol 2000 (Dow) | 2.5 ml |
| $d/H_2O$ | 1000 ml |
| | pH 7.0 |

**Medium 6**

| | |
|---|---|
| Dextrose | 2.0 % |
| Yeast Extract (Difco) | 2.0 |
| Casamino Acids (Difco) | 2.0 |
| $KNO_3$ | 0.2 |
| $MgSO_4 \cdot 7H_2O$ | 0.05 |
| NaCl | 0.05 |
| $FeSO_4 \cdot 7H_2O$ | 0.0025 |
| $CaCl_2 \cdot 2H_2O$ | 0.002 |
| $ZnSO_4 \cdot 7H_2O$ | 0.001 |
| $MnSO_4 \cdot H_2O$ | 0.0005 |
| $dH_2O$ | 1000 ml |
| | pH 7.0 with NaOH |

**Medium 7**

| | |
|---|---|
| Dextrose | 0.1 % |
| Soluble Starch (Fisher) | 1.0 |
| Beef Extract (Difco) | 0.3 |
| Yeast Autolysate (Ardamine pH Yeast Products) | 0.5 |
| NZ Amine Type E (Sheffield) | 0.5 |
| $MgSO_4 \cdot 7H_2O$ | 0.005 |
| $KH_2PO_4$ | 0.0182 |
| $Na_2HPO_4$ | 0.0190 |
| $CaCO_3$* | 0.05 |
| $dH_2O$ | 1000 ml |
| | pH 7.0 - 7.2 with NaOH |

* Added after pH adjustment

The fermentation can be conducted at temperatures ranging from 20°C to 40°C. For optimum results, it is most convenient to conduct these fermentations at a temperature in the range 24°C to 30°C, preferably 27° - 28°C. The pH of the nutrient medium can vary from 5.0 to 8.5, preferably 6.0 to 7.5.

Small-scale fermentations are conveniently carried out by placing suitable quantities of nutrient media in a flask using known sterile techniques, inoculating the flask with either spores or vegetative cellular growth of Nocardia autotrophica ATCC 35203, loosely stoppering the flask with cottonwool and permitting the fermentation to proceed in a constant temperature room of about 28°C on a rotary shaker at from 95 to 300 rpm for from 2 to 10 days. For larger-scale work, it is preferable to conduct the fermentation in suitable tanks provided with an agitator and a means of aerating the fermentation medium. The nutrient medium is made up in the tank and after sterilization is inoculated with a source of vegetative cellular growth of Nocardia autotrophica ATCC 35203. The fermentation is allowed to continue for from 1 to 8 days while agitating and/or aerating the nutrient medium at a temperature in the range of from 24°C to 37°C. The degree of aeration is dependent upon several factors, including the size of the fermentator and agitation speed. Generally the larger scale fermentations are agitated at about 95 to 500 RPM and 2 to 20 cubic feet per minute of air (1 to 10 litres/sec).

The separation of the novel compounds from the whole fermentation broth and their recovery is carried out by solvent extraction and application of chromatographic fractionations with various chromatographic techniques and solvent systems.

The compounds of the present invention have slight solubility in water, but are soluble in organic solvents. This property may be conveniently utilized to recover the compounds from the fermentation broth. Thus, in one recovery method, the whole fermentation broth is combined with approximately an equal volume of an organic solvent. While any organic solvent may be used, it is preferable to use a water-immiscible solvent such as ethyl acetate, methylene chloride or chloroform. Several extractions are usually desirable to achieve maximum recovery. The solvent removes the compounds of this present invention as well as other substances lacking their antiparastic activity. If the solvent is water-immiscible, the layers are separated and the organic solvent is evaporated under reduced pressure. If the solvent is water-miscible, it can be extracted with a water-immiscible solvent to separate the entrained water. This solvent can then be concentrated under reduced pressure. The residue is placed onto a chromatography column, preferably containing silica gel. The column retains the desired products and some impurities, but lets many of the impurities, particularly the nonpolar impurities, pass through. The column is washed with a moderately polar organic solvent such as methylene chloride or chloroform to further remove impurities, and is then washed with a mixture of methylene chloride or chloroform and an organic solvent of which acetone, ethyl acetate, methanol, and ethanol are preferred. The solvent is evaporated and the residue further chromatographed, e.g. using column chromatography, thin-layer chromatography, preparative-layer chromatography or high-pressure liquid chromatography, preferably reverse phase, with a chromatographic medium such as silica gel, aluminium oxide or a dextran gel with various solvents and combinations of solvents as the eluent. Thin-layer, high-pressure, liquid and preparative-layer chromatography may need to detect the presence of and to isolate the compound of the invention. The use of the foregoing and other known techniques will afford purified compositions containing the desired compound, the presence of which is determined by analysing the various chromatographic fractions for biological activity against selected parasites, or physico-chemical characteristics. The structure of the compounds has been determined by detailed analysis of their various spectral characteristics, in particular their nuclear magnetic resonance, mass, ultraviolet and infrared specta.

In the accompanying drawings, Figures 1, 2 and 3 are nuclear magnetic spectra of the compounds obtained in accordance with the present invention, which are identified below as compounds A, B and C respectively. They were originally recorded in $CDCl_3$ at ambient temperature on a Varian XL-400 NMR Spectrometer. Chemical shifts are shown in ppm relative to tetramethylsilane as an internal standard at zero ppm.

Based on these experimental data, the compounds of the present invention are believed to have the following structural formula based upon the analytical data given:

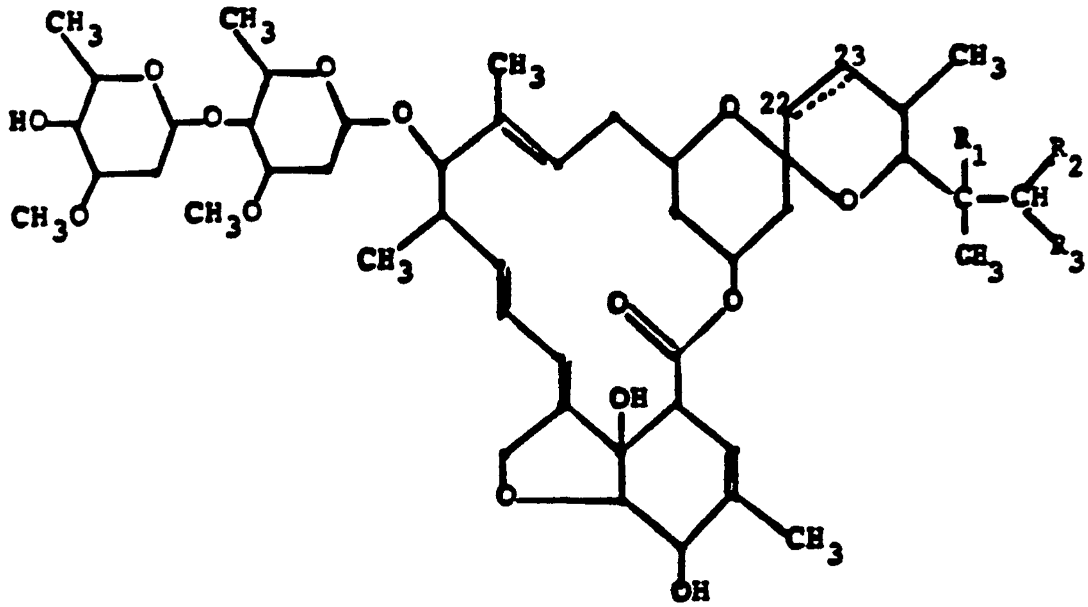

in which $R_1$, $R_2$, $R_3$ and the 22,23-linkage have the following values:

A: $R_1$ = H,$R_2$ = OH, $R_3$ = $CH_3$, 22,23-single bond
B: $R_1$ = H,$R_2$ = OH, $R_3$ = $CH_3$, 22,23-double bond
C: $R_1$ = OH, $R_2$ = H, $R_3$ = H, 22,23-double bond

The compounds are assigned the names: (A) 27-hydroxy-22,23-dihydro avermectin B1a; (B) 27-hydroxy avermectin B1a; and (C) 26-hydroxy avermectin B1b.

The molecular weights are:

| | Found | Calculated | For | Assignment |
|---|---|---|---|---|
| A | 890.5023 | 890.5028 | $C_{48}H_{74}O_{15}$ | M+ |
| B | 870.4767 | 870.4766 | $C_{48}H_{70}O_{14}$ | M+ |
| C | 856.4606 | 856.4609 | $C_{47}H_{68}O_{14}$ | M+ |

It has been found that each of the novel compounds of this invention possesses significant parasiticidal activity as an anthelmintic, insecticide and acricide, in human and animal health in agriculture.

The disease or group of diseases described generally as helminthiasis is due to infection of an animal host with parasitic worms known as helminths. Helminthiasis is a prevalent and serious economic problem in domesticated animals such as swine, sheep, horses, cattle, goats, dogs, cats and poultry. Among the helminths, the group of worms described as nematodes causes widespread and often times serious infection in various species of animals. The most common genera of nematodes infecting the animals referred to above are Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Heterakis, Toxocara, Ascaridia, Oxvuris, Ancylostoma, Uncinaria, Toxascaris and Parascaris. Certain of these, such as Nematodirus, Cooperia, and Oesophagostomum attack primarily the intestinal tract while others, such as Haemonchus and Ostertagia, are more prevalent in the stomach while other such as Dictyocaulus are found in the lungs. Still other parasites may be located in other tissues and organs of the body such as the heart and blood vessels, subcutaneous and lymphatic tissue and the like. The parasitic infections known as helminthiasis lead to anemia, malnutrition, weakness, weight loss, severe damage to the walls of the intestinal tract and other tissues and organs and, if left untreated, may result in death of the infected host. The compounds of this invention have unexpectedly high activity against these parasites, and in addition are also active against Dirofilaria in dogs, Nematospiroides, Syphacia, Asniculuris in rodents, arthropod ectoparasites of animals and birds such as ticks, mites, lice, fleas, blowfly, in sheep Lucilia sp., biting insects and such migrating dipterous larvae as Hypoderma sp. in cattle, Gastrophilus in horses, and Cuterebra sp. in rodents.

The instant compounds are also useful against parasites which infect humans. The most common genera of

parasites of the gastrointestinal tract of parasites of man are Ancylostoma, Necator, Ascaris, Strongyloides, Trichinella, Capillaria, Trichuris, and Enterobius. Other medically important genera of parasites which are found in the blood or other tissues and organs outside the gastro-intestinal tract are the filiarial worms such as Wuchereria, Brugia, Onchocerca and Dracunculus and extra intestinal stages of the intestinal worms Strongyloides and Trichinella. The compounds are also of value against arthropods parasitizing man, biting insects and other dipterous pests causing annoyance to man.

The compounds are also active against household pests such as the cockroach, Blatella sp., clothes moth, Tineola sp., carpet beetle, Attagenus sp. and the housefly Musca domestica.

The compounds are also useful against insect pests of stored grains such as Tribolium sp., Tenebrio sp. and of agricultural plants such as spider mites, (Tetranychus sp.), aphids (Acyrthiosiphon migratory orthopterans such as locusts and immature stages of insects living on plant tissue. The compounds are useful as a nematocide for the control of soil nematodes and plant parasites such as Meloidogyne spp. which may be of importance in agriculture.

These compounds may be administered orally in a unit dosage form such as a capsule, bolus or tablet, or as a liquid drench where used as an anthelmintic in mammals. The drench is normally a solution, suspension or dispersion of the active ingredient usually in water together with a suspending agent such as bentonite and a wetting agent or like excipient. Generally, the drenches also contain an antifoaming agent. Drench formulations generally contain from about 0.001 to 0.5 % by weight of the active compound. Preferred drench formulations may contain from 0.01 to 0.1 % by weight. The capsules and boluses comprise the active ingredient admixed with a carrier vehicle such as starch, talc, magnesium stearate, or dicalcium phosphate.

Where it is desired to administer the instant compounds in a dry, solid unit dosage form, capsules, boluses or tablets containing the desired amount of active compound usually are employed. These dosage forms are prepared by intimately and uniformly mixing the active ingredient with suitable finely divided diluents, fillers, disintegrating agents and/or binders such as starch, lactose, talc, magnesium stearate, vegetable gums and the like. Such unit dosage formulations may be varied widely with respect to their total weight and content of the antiparasitic agent depending upon factors such as the type of host animal to be treated, the severity and type of infection and the weight of the host.

When the active compounds are to be administered via an animal feedstuff, they are intimately dispersed in the feed or used as a top dressing or in the form of pellets which may then be added to the finished feed or optionally fed separately. Alternatively, the antiparasitic compounds of our invention may be administered to animals parenterally, for example, by intraruminal, intramuscular, intratracheal, or subcutaneous injection in which event the active ingredient is dissolved or dispersed in a liquid carrier vehicle. For parenteral administration, the active material is suitably admixed with an acceptable vehicle, preferably of the vegetable oil variety such as peanut oil, cotton seed oil and the like. Other parenteral vehicles such as organic preparations using solketal, glycerol, formal and aqueous parenteral formulations are also used. The active compounds are dissolved or suspended in the parenteral formulation for administration; such formulations generally contain from 0.005 to 5 % by weight of the active compound.

Although the antiparasitic agents of this invention finds its primary use in the treatment and/or prevention of helminthiasis, it is also useful in the prevention and treatment of diseases caused by other parasites, for example, arthropod parasites such as ticks, lice, fleas, mites and other biting insects in domesticated animals and poultry. They are also effective in treatment of parasitic diseases that occur in other animals including humans. The optimum amount to be employed for best results will, of course, depend upon the species of animal to be treated and the type and severity of parasitic infection or infestation. Generally, good results are obtained with our novel compounds by the oral administration of from about 0.001 to 10 mg per kg of animal body weight, such total dose being given at one time or in divided doses over a relatively short period of time such as 1 - 5 days. With the preferred compounds of the invention, excellent control of such parasites is obtained in animals by administering from about 0.025 to 0.5 mg per kg of body weight in a single dose. Repeat treatments are given as required to combat re-infections and are dependent upon the species of parasite and the husbandry techniques being employed. The techniques for administering these materials to animals are known to those skilled in the veterinary field.

When the compounds described herein is administered as a component of the feed of the animals, or dissolved or suspended in the drinking water, compositions are provided in which the active compound is intimately dispersed in an inert carrier or diluent. By inert carrier is meant one that will not react with the antiparasitic agent and one that may be administered safely to animals. Preferably, a carrier for feed administration is one that is, or may be, an ingredient of the animal ration.

Suitable compositions include feed premixes or supplements in which the active ingredient is present in relatively large amounts and which are suitable for direct feeding to the animal or for addition to the feed either directly or after an intermediate dilution or blending step. Typical carriers or diluents suitable for such compositions include, for example, distillers' dried grains, corn meal, citrus meal, fermentation residues, ground oyster shells, wheat shorts, molasses solubles, corn cob meal, edible bean mill feed, soya grits, crushed limestone and the like. The active compound is intimately dispersed throughout the carrier by methods such as grinding, stirring, milling or tumbling. Compositions containing from about 0.005 to 2.0 % by weight of the active compound are particularly suitable as feed premixes. Feed supplements, which are fed directly to the animal, contain from about 0.0002 to 0.3 % by weight of the active compounds.

Such supplements are added to the animal feed in an amount to give the finished feed the concentration of

active compound desired for the treatment and control of parasitic diseases. Although the desired concentration of active compound will vary depending upon the factors previously mentioned, the compounds of this invention are usually fed at concentrations of between 0.00001 to 0.002 % in the feed in order to achieve the desired anti-parasitic result.

In addition, where the instant compound is to be added to an animal's feed, it is possible to utilize the dried mycelial cake from the fermentation broth. The mycelia contain a preponderance of the activity and since the level of the activity of the mycelia can be determined,it can be added directly to the animal's feed.

The compounds of this invention have a broad spectrum of activity against many internal parasites at low dosage levels and in many different animals. At levels of about 2.5 mg per kg of animal body weight, concentrated mixtures of the instant compounds are fully active in sheep against Haemonchus contortus, Ostertagia circumcincta, Trichostrongylus axei, Trichostronaylus colubriformis, Cooperia spp., and Oesophagostomum columbianum. Similarly in cattle at dosages as low as 0.043 mg/kg the instant compounds are fully active against Ostertagia ostertage, Trichostrongylus axei, Trichostrongylus colubriformis, Oesophagostomum radiatum and Dictyocaulus viviparus. In addition, horses infected with bots (Gastrophilus intestinalis and Gastrophilus haemorrhoidalis), large and small strongylus and Oxyuris are successfully treated with 10 mg/kg (about 1 % active compound by weight) of a mixed concentrate of the instant compounds, and dogs infected with the microfilarial stage of heartworm (Dirofilaria immitis) are successfully treated with a single oral dose at 10 mg/kg (about 1 % active compound by weight) of a concentrate of the instant compound. In rodents, such as mice, infections of Syphacia, Nematospiroides and Aspiculuris are successfully treated by the oral administration of the instant compound or of the concentrate obtained from the extraction of the mycelia.

The compounds of this invention are also useful in combatting agricultural pests that inflict damage upon crops while they are growing or while in storage. The compounds are applied using known techniques as sprays, dusts, emulsions and the like, to the growing or stored crops to effect protection from such agricultural pests.

The anthelmintic activity of the instant compounds may be determined by orally administering via the feed, a sample of the individual compound, a concentrated extract, and the like to a mouse which had been infected 3 days earlier with Nematospiroides dubius. At 11, 12 and 13 days after the initiation of the medication, the feces of the mouse are examined for N. dubius eggs, and on the next day the mouse is sacrificed and the number of worms present in the proximal portion of the small intestine are determined. An active compound is observed when there is a significant reduction of egg and worm counts when compared to infected, unmedicated controls.

The following examples are being provided in order that the instant invention may be more fully understood. Such examples are not to be construed as being limitative of the invention.

**Example 1**
**Transformation Methodology**

| | | |
|---|---|---|
| Media: | Seed Medium A | g/l |
| | Dextrose | 4.0 g |
| | Nutrient Broth | 4.0 g |
| | Yeast Extract | 4.0 g |
| | Malt Extract | 10.0 g |
| | 1000 ml distilled $H_2O$ pH 7.3 | |
| | Slant Medium B | |
| | Medium A plus Agar | 20.0 g |
| | Transformation Medium C | |
| | Same as Medium A, plus | |
| | substrate at | 0.25 g |

A lyophile tube was aseptically opened and grown in seed Medium A (20 ml in a 250 ml 3-baffle Erlenmyer flask) for 48 hours on a rotary shaker (220 rpm) at 27°C.

This seed was then used to inoculate slants (Medium B), transformation flasks (Medium C), and to prepare frozen vials for future studies.

The substrate was added post sterilization and prior to inoculation. Methanol was used to solubilize the substrate for filter sterilization and addition. The transformation flasks (40 ml Medium C in 250 ml 3-baffle Erlenmyer flask) were incubated for 7 days with agitation (220 rpm) at 27°C. Following incubation, the whole broths were extracted as follows:

**Extraction Methodology**

a. 50 ml methylene chloride were added to 40 ml whole broth and mechanically agitated for 15 minutes. The emulsion was broken by centrifugation and methylene chloride separated. Step "a" was repeated 3 times.

b. The pooled methylene chloride extracts were taken to dryness under vacuum.

c. The dried methylene chloride fraction was solubilized with 25 ml (x3) ethanol/0.1 M $K_2HPO_4$, pH 7.0 (40/60). Three extracts pooled.

d. The phosphate buffer: ethanol fraction was extracted with 25 ml cyclohexane (x3) to remove the residual substrate. The cyclohexane fractions were pooled and taken to dryness under vacuum. The residue was solubilized with a known volume of methanol, dried with anhydrous $Na_2SO_4$ and, where appropriate, total radioactivity determined by scintillation counting.

e. The phosphate buffer: ethanol fraction previously extracted with cyclohexane, was then extracted with 25 ml methylene chloride (x3) to separate the altered substrate. The methylene chloride fractions were pooled and taken to dryness under vacuum. The residue was solubilized with a known volume of methanol, dried with anhydrous $Na_2SO_4$ and total, where appropriate, radioactivity determined by scintillation counting.

f. All organic fractions were submitted for HPLC analysis to determine and isolate non-substrate avermectins.

**Specific Example A:**

| | |
|---|---|
| Culture: | Nocardia autotrophica MA6181 ATCC 35203 |
| Substrate: | 1 mg $^3$H-22,23, dihydro avermectin B1a |
| Sample: | Methylene chloride Ext. (A) 1.1 x $10^6$ CPM Total |
| Sample: | Cyclohexane Ext. (B) 4.0 x $10^6$ CPM Total |

**Specific Example B:**

| | |
|---|---|
| Culture: | Nocardia autotrophica MA6181 ATCC 35203 |
| Substrate: | 25 mg 22,23, dihydro avermectin B1a. Twenty five flasks pooled and extracted for product isolation and identification. |
| Sample: | Cyclohexane Ext. (C) |
| Sample: | Methylene chloride Ext. (D) |

**Example 2**

The final methylene chloride extract residue, sample D from Example 1B was dissolved in 400 microliters of 85/15 v/v methanol/water, filtered and the filtrate subjected to preparative HPLC chromatography on a Dupont Zorbax ODS reverse phase $C_{18}$ column 0.94 x 25 cm, at room temperature, using a solvent system of 85/15 v/v methanol/water at a flow rate of 4 ml/minute. The effluent stream was monitored at 243 nm using an LDC Spectromonitor II with a one mm path length cell at a setting of 0.64 AUFS, and a Spectra-Physics SP4100 computing integrator. Eleven fractions were collected. Fraction ten, 22.5 minutes to 25.5 minutes, was concentrated to dryness. The residue was taken up in 1 ml of methanol and labeled Sample E.

**Example 3**

The cyclohexane extract residue, sample C from Example 1B was dissolved in 700 mcl of 85/15 v/v methanol/water, filtered and the filtrate subjected to preparative HPLC chromatography on a Dupont Zorbax ODS reverse phase $C_{18}$ column 0.94 x 25 cm, at room temperature using a solvent system of 85/15 v/v methanol/water at a flow rate of 4 ml/minute. The effluent stream was monitored at 243 nm using an LDC

Spectromonitor II with a one mm path length cell at a setting of 0.32 AUFS, and a Spectra-Physics SP4100 computing integrator. Eleven fractions were collected. Fraction three, 23 minutes to 25.5 minutes, was concentrated to dryness. The residue was taken up in one-half ml of methanol and labeled Sample F.

Samples E and F were combined in 5 ml of methanol and labeled G. Ultraviolet quantitation of solution carried out as follows: assaying at a dilution of 1 : 5 in methanol.

$$\text{Conc X} = \frac{\text{O.D. 244 nm X 10 X dilution}}{0.365} \text{ X volume}$$

$$\text{Conc X} = \frac{2.190 \text{ X } 10 \text{ X } 5}{0.365} \text{ X } 5 = 1{,}500 \text{ mcg}$$

**Example 4**

Sample G from Example 3 was concentrated to dryness and the residue taken up in 200 mcl of methanol and subjected to preparative HPLC chromatography on a Dupont Zorbax ODS $C_{18}$ reverse phase column 0.94 x 25 cm at room temperature using a solvent system of 85/15 v/v methanol/water at a flow rate of 4 ml/minute for thirty-four minutes followed by a gradient of 85 % methanol to 100 % methanol over five minutes at 4 ml/minute and maintaining at 100 % methanol for seventy-six minutes.

The effluent stream was monitored at 243 nm using an LDC Spectromonitor II with a one mm path length cell at a setting of 0.32 AUFS, and a SpectraPhysics SP4100 computing integrator. Seven fractions were collected. Fraction six, 27 minutes to 29 minutes was concentrated to dryness. The residue was taken up in 10 ml of methanol and diluted 1 : 5 with methanol for ultra-violet quantitation. Sample labeled H.

$$\text{Conc. X} = \frac{\text{O.D. 244 nm X 10 X dilution}}{0.365} \text{ X volume}$$

$$\text{H} = \frac{0.450 \text{ X } 10 \text{ X } 5}{0.365} \text{ X } 10 = 616 \text{ mcg}$$

Sample H assigned the structure 27-hydroxy-22,23-dihydro avermectin B1a. Figure 1 is the nuclear magnetic resonance spectrum for this compound.

**Example 5**

The transformation and extraction methodology of Example 1 was repeated for the following Specific Examples.

**Specific Example C**

| | |
|---|---|
| Culture: | <u>Nocardia autotrophica</u> MA-6181, ATCC-35203 |
| Substrate: | 2 mg Avermectin B1a |
| Sample: | Methylene chloride Ext. (E) |

**Specific Example D**

| | |
|---|---|
| Culture: | <u>N. autotrophica</u> MA-6181, ATCC-35203 |
| Substrate: | 1 mg Avermectin B1a |
| Sample: | Methylene chloride Ext. (E) |

**Specific Example E**

| | |
|---|---|
| Culture: | <u>N. autotrophica</u> MA-6181, ATCC-35203 |

Substrate: 45 mg Avermectin B1a. Forty-five flasks pooled for product isolation and identification.
Sample: Methyl chloride Ext. (E)

**Specific Example F**

Culture: N. autotrophica MA-6181, ATCC-35203
Substrate: 50 mg Avermectin B1a. Fifty flasks pooled for product isolation and identification
Sample: Methylene chloride Ext. (E)

**EXAMPLE 6**

Sample E from Specific Example E was concentrated to 0.5 ml in methanol and filtered. The filter was washed with 0.1 ml of methanol and the filtrate and wash combined. The combined filtrate and wash was subjected to preparative HPLC chromatography on a Dupont Zorbax ODS C18 column 0.94 x 25 cm maintained at room temperature. The chromatography was carried out using a solvent of 80/20 v/v methanol/water at a flow rate of 4 ml/minute. The effluent stream was monitored at 243 nm using an L.D.C. Spectro-Monitor-II with a lmm path length cell and a setting of 0.64 AUFS. Thirty-one fractions were collected based on the ultra-violet trace. Selected fractions were concentrated to dryness and taken up in 1 ml of methanol for quantitation. The samples were quantitated using an analytical HPLC system of 80/20 v/v methanol/water at 1 ml/minute and a Dupont Zorbax ODS C18 column 0.46 x 25 cm maintained at 27°C, monitoring the effluent at 243 nm. The sample concentrations were calculated as follows:

$$\text{Concentration of X} = \frac{\text{Total area counts of X}}{\text{area counts/mcg avermectin-B1a}}$$

Fractions 13 and 14 were found to contain 1.53 and 0.24 mg respectively of the desired compound. Fractions 13 and 14 were combined and labeled Sample E-1 which was identified as 27-hydroxyavermectin-B1a. Figure 2 is the nuclear magnetic resonance spectrum for this compound.

**Example 7**

The transformation and extraction methodology of Example 1 was repeated for the following Specific Examples:

**Specific Example G**

Culture: Nocardia autotrophica MA-6181, ATCC-35203
Substrate: 2 mg Avermectin B1b
Sample: Methylene chloride Ext. (F)

**Specific Example H**

Culture: N. autotrophica MA-6181, ATCC-35203
Substrate: 1 mg Avermectin B1b
Sample: Methylene chloride Ext. (F)

**Specific Example I**

Culture: N. autotrophica MA-6181, ATCC-35203

Substrate: 45 mg Avermectin B1b. Forty-five flasks pooled for product isolation and identification.
Sample: Methylene chloride Ext. (F)

**Example 8**

Sample F from Specific Example III was concentrated to dryness and the residue taken up in 0.5 ml of methanol. This solution was filtered and the filtrate subjected to preparative HPLC chromatography on a Dupont Zorbax ODS C18 column 0.94 x 25 cm at room temperature. The chromatography was carried out at 4 ml/minute using the following gradient developed by an DuPont 8800 gradient controller.

Gradient: 75/25 methanol/water for 68 minutes than a linear gradient over one minute to 77/23 methanol/water, hold for 23 minutes, then a linear gradient over one minute to 79/21 methanol water, hold for 30 minutes then linear gradient to 100 % methanol over 10 minutes, hold for 30 minutes.

The effluent stream was monitored at 243 nm using an L.D.C. Spectro-Monitor-II with a 1 mm path length cell and a setting of 1.28 AUFS. Thirty fractions were collected based on the ultra-violet trace. Selected fractions were concentrated to dryness and taken up in 1 ml of methanol for quantitation. The selected fractions were quantitated using an analytical HPLC system of 80/20 v/v methanol/water at 1 ml/minute and a DuPont Zorbax ODS C18 column 0.46 x 25 cm maintained at 27°C, monitoring the effluent at 243 nm. The sample concentrations were calculated as follows:

$$\text{Conc X} = \frac{\text{area counts of X}}{\text{area counts/mcg avermectrin-B1a}} \times \frac{\text{M.W. X}}{\text{M.W. B1a}}$$

Fraction 10 contained 1.3 mg of 26-hydroxy-avermectin-B1b and fraction 23 contained 0.61 mg of 3"-O-desmethyl-avermectin-B1b. Figure 3 is the nuclear magnetic resonance spectrum for 26-hydroxy-avermectin B1b.

## Claims

1. A compound having the formula:

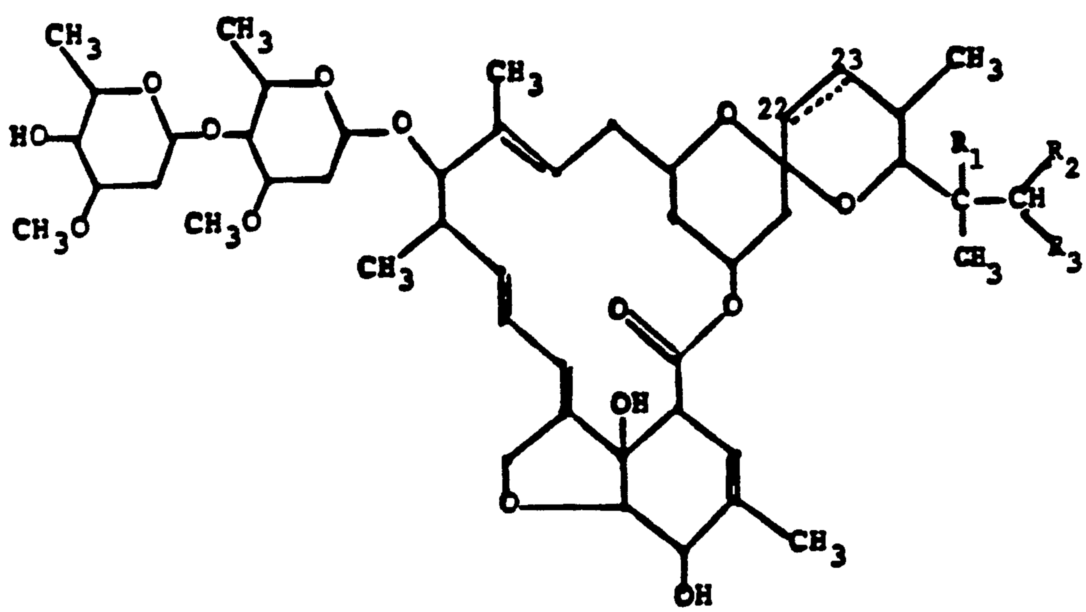

in which $R_1$, $R_2$, $R_3$ and the 22,23-linkage have the following values:
A: $R_1$ = H,$R_2$ = OH, $R_3$ = $CH_3$, 22,23-single bond
B: $R_1$ = H,$R_2$ = OH, $R_3$ = $CH_3$, 22,23-double bond
C: $R_1$ OH, $R_2$ = H, $R_3$ = H, 22,23-double bond

2. A process for the preparation of a compound as claimed in Claim 1 that comprises fermenting 22,23-dihydro-avermectin B1a for compound A, avermectin B1a for compound B or avermectin B1b for compound C, in a source of carbon, nitrogen, and inorganic salts, using the microorganism Nocardia autotrophica ATCC 35203, and isolating the resulting compound from the fermentation medium.

3. A process as claimed in Claim 2 in which the compound is isolated by solvent extraction followed by at

least two chromatographic extraction steps.

4. A compound as claimed in Claim 1 for use in the treatment of parasitic diseases in animals.

5. A composition useful for the treatment of parasitic diseases comprising an inert excipient or carrier and a compound as claimed in Claim 1.

**Patentansprüche**

1. Verbindung mit der Formel:

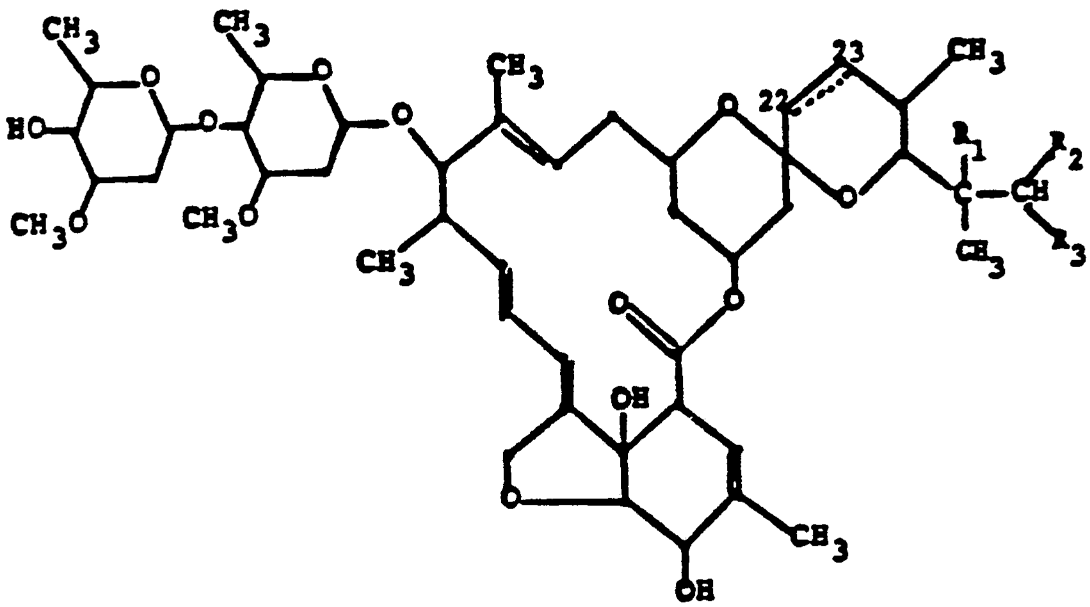

worin $R_1$, $R_2$, $R_3$ und die 22,23-Bindung die folgenden Bedeutungen haben:

A: $R_1$ = H, $R_2$ = OH, $R_3$ = $CH_3$, 22,23-Einfachbindung

B: $R_1$ = H, $R_2$= OH, $R_3$ = $CH_3$, 22,23-Doppelbindung

C: $R_1$ = OH, $R_2$ = H, $R_3$ = H, 22,23-Doppelbindung.

2. Verfahren zur Herstellung einer Verbindung, wie in Anspruch beansprucht, welches die Fermentierung von 22,23-Dihydroavermectin B1a für Verbindung A, von Avermectin B1a für Verbindung B oder von Avermectin B1b für Verbindung C in einer Quelle für Kohlenstoff, Stickstoff und anorganische Salze unter Verwendung des Mikroorganismus Nocardia autotrophica ATCC 35203 und die Isolierung der resultierenden Verbindung aus dem Fermentierungsmedium umfaßt.

3. Verfahren, wie in Anspruch 2 beansprucht, worin die Verbindung durch Lösungsmittelextraktion, gefolgt von wenigstens zwei chromatographischen Extraktionsschritten, isoliert wird.

4. Verbindung, wie in Anspruch beansprucht, zur Verwendung bei der Behandlung von parasitären Krankheiten in Tieren.

5. Zur Behandlung von parasitären Krankheiten geeignete Zusammensetzung, welche einen inerten Exzipienten oder Träger und eine Verbindung, wie in Anspruch beansprucht, umfaßt.

**Revendications**

1. Composé répondant à la formule:

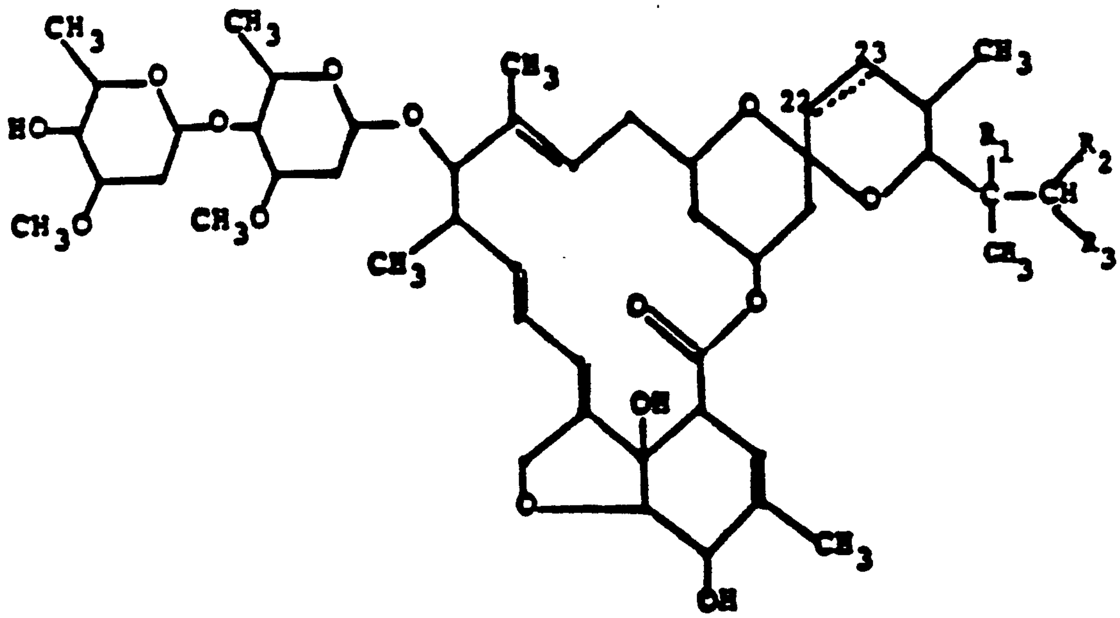

dans laquelle $R_1$, $R_2$, $R_3$ et la liaison 22,23 ont les significations suivantes:

A: $R_1 = H$, $R_2 = OR$, $H_3 = CH_3$, simple liaison 22,23

B: $R_1 = H$, $R_2 = OH$, $R_3 = CH_3$, double liaison 22,23

C: $R_1 = OH$, $R_2 = H$, $R_3 = H$, double liaison 22,23

2. Procédé pour la préparation d'un composé selon la revendication 1, qui comprend la fermentation de la 22,23-dihydro-avermectine B1a pour le composé A, de l'avermectine B1a pour le composé B ou de l'avermectine B1b pour le composé C dans une source de carbone, d'azote et des sels minéraux en utilisant le micro-organisme Nocardia autotrophica ATCC 35203, et l'isolement du composé obtenu à partir du milieu de fermentation.

3. Procédé selon la revendication 2, dans lequel le composé est isolé par extraction avec un solvant, suivie d'au moins deux stades d'extraction chromatographique.

4. Composé selon la revendication 1 pour l'utilisation dans le traitement des maladies parasitaires chez les animaux.

5. Composition utile pour le traitement des maladies parasitaires comprenant un excipient ou support inerte et un composé selon la revendication 1.

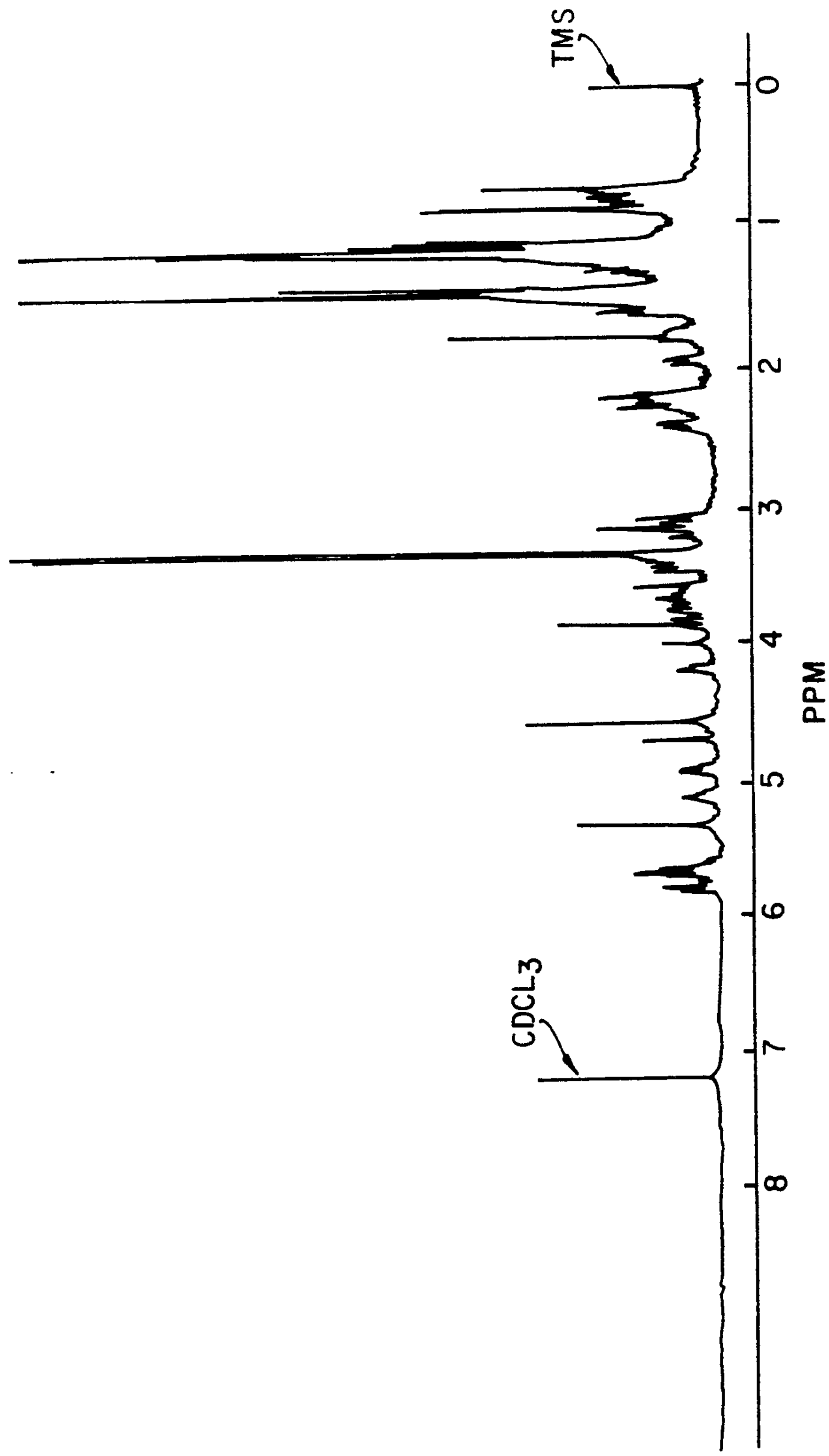


FIG.1

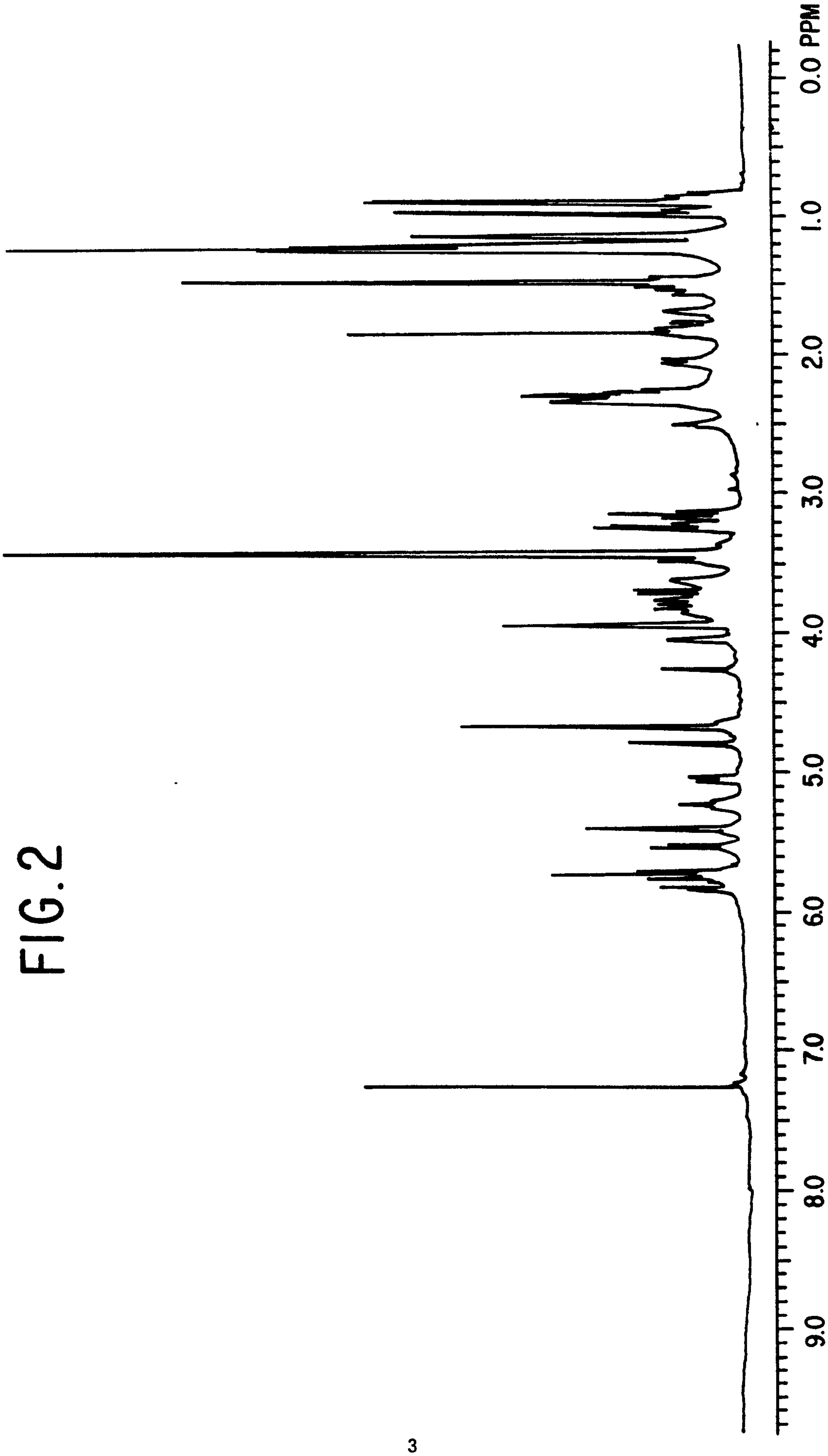


FIG. 2

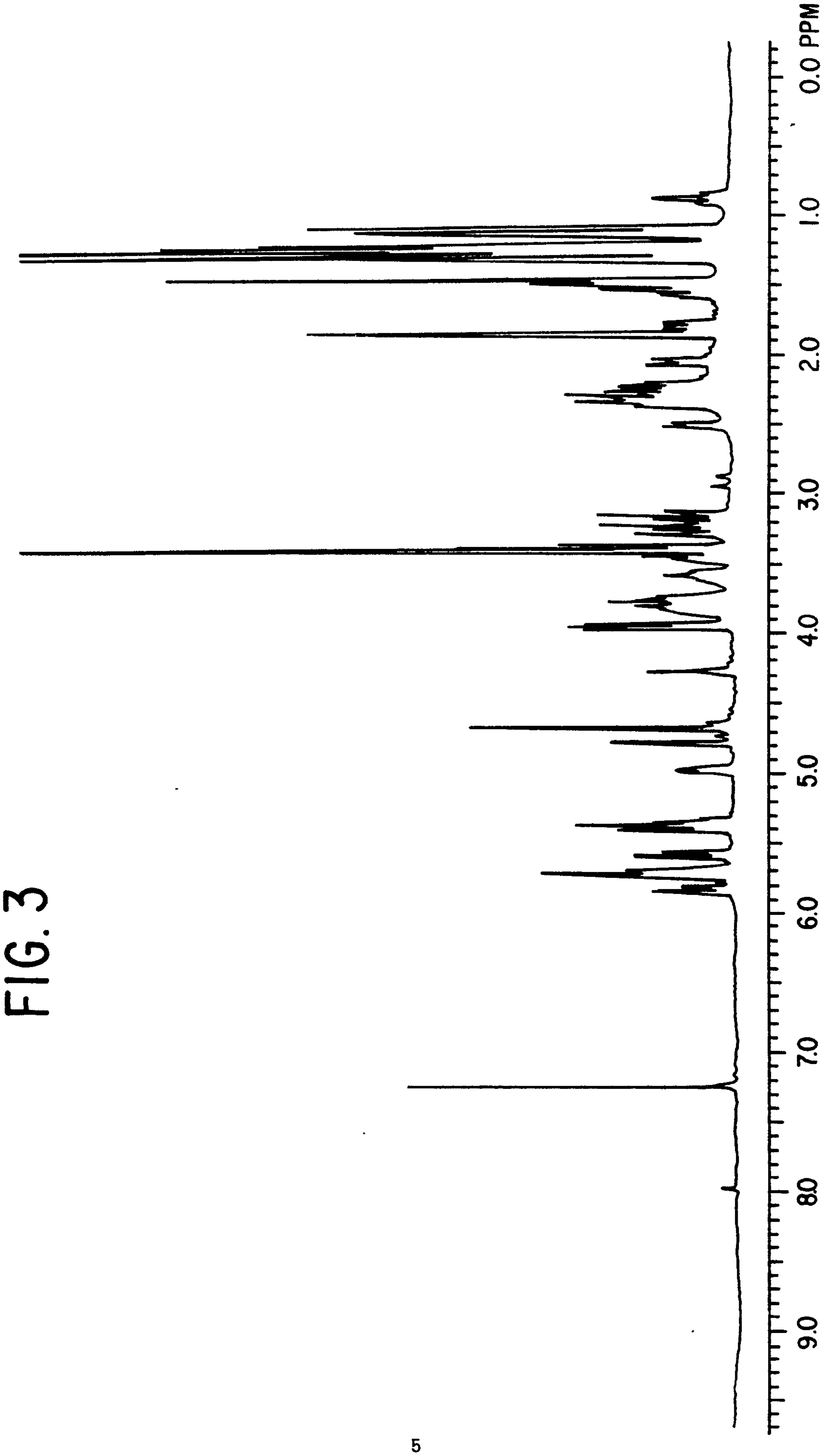


FIG. 3